# EUROPÄISCHE PATENTSCHRIFT

(11) **EP 1 091 781 B1**
(45) Veröffentlichungstag und Bekanntmachung des Hinweises auf die Patenterteilung: **29.10.2003**
(21) Anmeldenummer: 98963381.3
(22) Anmeldetag: 26.11.1998
(51) Int. Cl.: A61M 16/20, A61M 16/08

(54) **VORRICHTUNG ZUM ENTFERNEN VON SPUTUM AUS EINEM LUFTRÖHRENKATHETER**
DEVICE FOR REMOVING SPUTUM FROM A TRACHEAL CATHETER
DISPOSITIF POUR ENLEVER DES MUCOSITES SE TROUVANT DANS UN CATHETER TRACHEEN

(30) Priorität: 28.06.1998 DE 29811374 U; 24.08.1998 DE 19838370
(43) Veröffentlichungstag der Anmeldung: 18.04.2001
(73) Patentinhaber: Enzinger, Alfred, 90469 Nürnberg (DE)
(72) Erfinder: Enzinger, Alfred, 90469 Nürnberg (DE)
(74) Vertreter: Tergau, Enno, Dipl.-Ing.
(86) Internationale Anmeldenummer: DE9803473
(87) Internationale Veröffentlichungsnummer: WO00000246

(56) Entgegenhaltungen:
- DE-U- 8 625 936
- FR-A- 1 552 128
- US-A- 3 603 313
- US-A- 5 433 195
- US-A- 5 765 557

## Beschreibung

Die Erfindung betrifft eine Vorrichtung zum Entfernen von Sputum (durch Husten entleerter Auswurf aus den Atmungsorganen) aus einem in die Luftröhre eines Patienten eingesetzten Luftröhrenkatheter.

Aus der US-PS 5,765,557 ist es bekannt, daß sich bei Patienten, denen ein Luftröhrenkatheter in die Luftröhre eingesetzt ist, eine erhöhte Menge von Sputum in der Lunge ansammelt, das durch regelmäßiges Absaugen entfernt werden muß. Diese bekannte Technik ist jedoch äußerst unvorteilhaft und erhöht in beträchtlichem Maße die Gefahr von Infektionen. Deshalb ist nach diesem US-Patent eine Vorrichtung zum Steuern des Luftstroms innerhalb des Patienten vorgesehen, um ein Entfernen des Sputums aus dem Innern des Patienten ohne Ansaugen zu erleichtern. Diese Vorrichtung enthält einen in das Innere des Patienten einsetzbaren Kanal mit einem Pfad zum Einatmen und einem getrennten Pfad zum Ausatmen sowie eine Luftstromsteuerung innerhalb der Vorrichtung zum Steuern der durch den Einatmungspfad strömenden Atemluft zum Ausdehnen der Lunge des Patienten und um der auszuatmenden Luft zu ermöglichen, durch den getrennten Ausatmungspfad zu strömen, um das Sputum aus dem Patienten durch diesen Ausatmungspfad durch die Kraft der von der Lunge ausgestoßenen Atemluft zu entfernen.

Diese innerhalb des Patienten vorgesehene Vorrichtung zum Steuern des Luftstroms kann von außen nicht überwacht werden, so daß deren einwandfreie Funktion nur vermutet werden kann. Die bei einer Funktionsstörung notwendige Hilfe kommt dann oft zu spät. Deshalb hat sich diese Vorrichtung im praktischen Betrieb nicht durchgesetzt.

Anstelle dieser Vorrichtung wird deshalb in der Praxis nach wie vor ein aus der DE 35 24 126 A1 bekannter Luftröhrenkatheter verwendet, dessen vorderes Ende beispielsweise durch einen Luftröhrenschnitt in die Luftröhre eingesetzt werden kann, während an seinem hinteren, aus der Luftröhre ragenden Ende ein Verbindungsstück angeordnet ist, auf das beispielsweise ein Luftschlauch zum Verbinden mit einer Beatmungsmaschine oder ein Luftfilter aufsteckbar ist, über das der Patient gefilterte, also keimfreie Atemluft aufnehmen kann.

Es hat sich jedoch gezeigt, daß in vielen Fällen der Husten so stark ist, daß das aufgesteckte Luftfilter in kurzer Zeit, mitunter schon nach weniger als zehn Minuten, mit Sputum derart verstopft ist, daß der Patient keine Luft mehr bekommt und qualvoll gegen das Ersticken ankämpfen muß, wenn das verstopfte Filter nicht sofort entfernt und durch ein neues ersetzt wird. Da in vielen Fällen die betroffenen Patienten gelähmt sind, beispielsweise durch Schlaganfälle oder durch einen Gehirnschlag, können sie das Filter nicht selbst entfernen, sondern sind auf die Hilfe des Pflegepersonals angewiesen. Da bekanntlich auch im Pflegedienst großer Personalmangel herrscht, sind die Patienten häufig auf die zusätzliche Hilfe durch Verwandte und Bekannte angewiesen. In der Realität kommt es auch häufig vor, daß das Filter einfach weggelassen wird, weil das Personal wegen Zeitmangels dieses nicht ständig wechseln kann, so daß der Patient ungefilterte Luft einatmen muß, was jedoch die Gefahr von gefährlichen oder gar tödlichen Infektionen stark erhöht.

Der Erfindung liegt die Aufgabe zugrunde, den bekannten Luftröhrenkatheter derart zu ergänzen, daß das Sputum automatisch von dem Luftfilter ferngehalten und problemlos entfernt wird, so daß die qualvollen und schmerzhaften Erstickungsanfälle der Patienten vermieden werden und die Gefahr des Erstickungstodes gebannt ist, selbst dann, wenn der Patient über längere Zeit, etwa mehrere Stunden, ohne Aufsicht gelassen wird.

Diese Aufgabe wird gemäß Anspruch 1 gelöst durch ein Gehäuse
- mit einer ersten Öffnung, die mit dem aus der Luftröhre ragenden Ende des Luftröhrenkatheters verbindbar ist,
- mit einer zweiten Öffnung für den Austausch ausgeatmeter Luft gegen gefilterte Frischluft und
- mit einer dritten Öffnung für den Abfluß des Sputums.

Gemäß der Erfindung ist ein durch die Atemluft steuerbares Ventil vorgesehen, das die dritte Öffnung während des Ausatmens zum Abfluß des Sputums öffnet und während des Einatmens verschließt. Dabei hat das Gehäuse zweckmäßig die Form eines Zylinders, wobei die erste Öffnung in der Grundfläche des Zylinders und die zweite und die dritte Öffnung jeweils in dessen Mantelfläche im wesentlichen gegenüberliegend angeordnet sind und wobei die Öffnungen mit zylindrisch geformten Anschlußstücken versehen sind.

Zum Verbinden der Vorrichtung mit dem Luftröhrenkatheter und mit anderen Apparaten ist weiterhin vorgesehen, daß die Innenwand des Anschlußstückes der ersten Öffnung konisch geformt und auf das aus der Luftröhre ragende Ende des Luftröhrenkatheters aufsteckbar ist, während das Anschlußstück der in der Gebrauchslage vorzugsweise nach oben gerichteten zweiten Öffnung eine konisch geformte Außenwand aufweist, auf die ein Luftfilter für die einzuatmende Luft aufsteckbar ist, wobei ferner das Anschlußstück der in der Gebrauchslage vorzugsweise nach unten gerichteten dritten Öffnung eine konisch geformte Außenwand aufweist, auf die ein Auffangbeutel zum Auffangen des Sputums oder ein Schlauch für den Abfluß des Sputums zu einem Auffangbeutel aufsteckbar ist.

Die Ausgestaltung des Ventils zum Öffnen und Schließen der dritten Öffnung kann im einfachsten Fall vorsehen, daß es aus einer durch die Atemluft gesteuerten schwenkbaren Klappe besteht, die die dritte Öffnung während des Ausatmens durch den Druck der ausgeatmeten Luft und gegen den Druck einer Feder, ihres Eigengewichts oder eines Zusatzgewichts zum Abfluß des Sputums öffnet und während des Einatmens durch den Druck der Feder, ihres Eigengewichts oder eines Zusatzgewichts verschließt.

Eine andere Alternative für das Öffnen und Schließen der dritten Öffnung sieht vor, daß das Ventil aus einem durch die Atemluft gesteuerten Kolben besteht, der die dritte Öffnung während des Ausatmens durch den Druck der ausgeatmeten Luft und gegen den Druck einer Feder, seines Eigengewichts oder eines Zusatzgewichts zum Abfluß des Sputums öffnet und während des Einatmens durch den Druck der Feder, seines Eigengewichts oder eines Zusatzgewichts verschließt.

Weitere vorteilhafte Ausgestaltungen der Erfindung sind in den weiteren Unteransprüchen angegeben.

Die mit der Erfindung erzielten Vorteile bestehen insbesondere darin, daß das Sputum automatisch von dem Luftfilter ferngehalten und problemlos entfernt wird, so daß dem Patienten die qualvollen Erstickungsanfälle erspart bleiben und die Gefahr des Erstickungstodes gebannt ist. Die einwandfreie Funktion der Vorrichtung kann jederzeit und auf einen Blick überprüft werden, so daß bei einer etwaigen Funktionsstörung die notwendige Hilfe schnell geleistet werden kann.

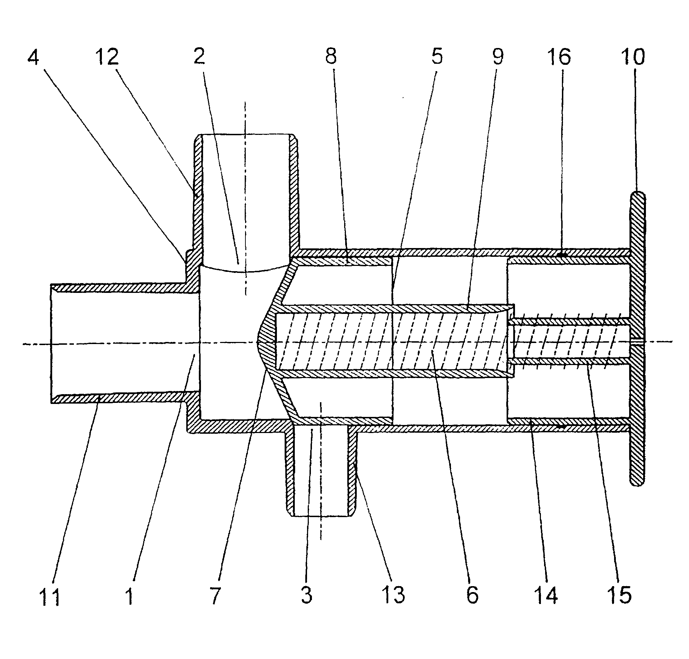

Ein Ausführungsbeispiel der Erfindung ist in der Zeichnung dargestellt und wird im folgenden näher beschrieben.

Die erfindungsgemäße Vorrichtung besteht aus einem Gehäuse 4, das die Form eines Zylinders aufweist, dessen Symmetrieachse in der Gebrauchslage vorzugsweise waagerecht verläuft, wobei eine erste Öffnung 1 in der Grundfläche des Zylinders angeordnet ist, während zwei weitere Öffnungen 2, 3 jeweils in der Mantelfläche angeordnet sind. Alle drei Öffnungen 1, 2, 3 sind mit zylindrischen Anschlußstücken 11, 12, 13 versehen.

Die Innenwand des Anschlußstückes 11 der ersten Öffnung 1 ist konisch geformt, so daß dieses Anschlußstück 11 auf das aus der Luftröhre ragende Ende des Luftröhrenkatheters aufgesteckt werden kann. Das Anschlußstück 12 der in der Gebrauchslage vorzugsweise nach oben gerichteten zweiten Öffnung 2 weist eine konisch geformte Außenwand auf, auf die ein Luftfilter zum Reinigen und Entkeimen der einzuatmenden Luft aufsteckbar ist. Das Anschlußstück 13 der in der Gebrauchslage vorzugsweise nach unten gerichteten dritten Öffnung 3 weist eine konisch geformte Außenwand auf, auf die ein Auffangbeutel zum Auffangen des Sputums oder ein Schlauch für den Abfluß des Sputums zu einem derartigen Auffangbeutel aufgesteckt werden kann.

Das Ventil zum Öffnen und Schließen der dritten Öffnung 3 besteht aus einem durch die Atemluft gesteuerten Kolben 5, der eine kegelförmige Grundfläche 7 und eine zylindrische Mantelfläche 8 aufweist und in dem zylindrischen Gehäuse 4 leicht gleitend koaxial gelagert ist. Dabei wird die dritte Öffnung 3 im verschlossenen Zustand von der Mantelfäche 8 überdeckt.

Innerhalb der zylindrischen Mantelfläche 8 des Kolbens 5 ist ein koaxial verlaufendes zylindrisches Rohr 9 einstückig angeformt, das einen großen Teil einer als Schraubenfeder ausgebildeten Feder 6 in sich aufnimmt, deren anderer Teil sich auf einer Verschlußplatte 10 abstützt, die eine an der der ersten Öffnung 1 gegenüberliegenden Seite des zylindrischen Gehäuses 4 vorhandene vierte Öffnung abdeckt. An dieser Verschlußplatte 10 sind zwei koaxial zueinander angeordnete zylindrische Rohre 14, 15 einstückig angeformt, wobei das äußere Rohr 14 an der Innenwand des Gehäuses 4 anliegend in das Gehäuse 4 einführbar und in eine Innenwandrille 16 einrastbar ist, während das innere Rohr 15 in die Feder 6 führend eingreift.

Die Bewegung des Kolbens 5 wird durch die Atemluft im Zusammenspiel mit der Feder 6 derart gesteuert, daß er während des Ausatmens durch den Druck der ausgeatmeten Luft und gegen den geringeren Druck der Feder 6 in Richtung zu der vierten Öffnung hin verschoben wird und dadurch die mit seiner zylindrischen Mantelfläche abgedeckte, in der Gebrauchslage nach unten gerichtete dritte Öffnung 3 freigibt, so daß das Sputum abfließen kann, während beim Einatmen der Kolben 5 durch den Druck der Feder 6 und durch den Wegfall des Gegendrucks der Atemluft in entgegengesetzter Richtung bewegt wird und die dritte Öffnung 3 wieder verschließt, so daß keine ungefilterte Luft über diese Öffnung 3 eingeatmet werden kann. Die Atemluft strömt in jeder Phase des Atmungsvorganges immer nur durch die zweite Öffnung 2 und über das auf das Anschlußstück 12 aufgesetzte Luftfilter.

Das zylindrische Gehäuse 4 mit seinen Anschlußstücken 11, 12, 13 und/oder die in seinem Inneren angeordneten Teile 5, 6, 7, 8, 9, 10, 14, 15, 16 sollten zumindest teilweise zweckmäßig aus Kunststoff bestehen, vorzugsweise aus durchsichtigem Kunststoff, damit der Verlauf des Sputums im Inneren des Gehäuses jederzeit kontrolliert werden kann, um rechtzeitig eingreifen zu können, wenn wider Erwarten der Kolben durch festere Bestandteile des Sputums in seiner freien Beweglichkeit behindert werden sollte.

Der Erfinder der vorliegenden Erfindung hat mit einem Prototyp der erfindungsgemäßen Vorrichtung über längere Zeit hinweg die einwandfreie Funktion an einem durch einen Gehirnschlag gelähmten, ihm nahestehenden Patienten unter Aufsicht von Ärzten und Pflegepersonal erprobt. Dabei war eine Reinigung der Vorrichtung über Tage hinweg nicht erforderlich, während ohne diese Vorrichtung das verstopfte Filter in akuten Fällen im Durchschnitt alle zehn bis 15 Minuten ausgetauscht werden muß. Die Vorrichtung fand großen Beifall bei den Ärzten und dem Pflegepersonal. Der Einsatz in allen Intensivstationen und Rehabilitationszentren ist daher aus humanitären Gründen dringend geboten.

## Patentansprüche

1. Vorrichtung zum Entfernen von Sputum aus einem in die Luftröhre eines Patienten eingesetzten Luftröhrenkatheter, bestehend aus einem zylindrischen Gehäuse (4) mit einer ersten, in der Grundfläche des Zylinders angeordneten Öffnung (1), die mit dem aus der Luftröhre ragenden Ende des Luftröhrenkatheters verbindbar ist, mit einer zweiten Öffnung (2), über die ausgeatmete Luft gegen gefilterte Frischluft austauschbar ist, und mit einer dritten, in der Mantelfläche des Zylinders angeordneten Öffnung (3), über die das Sputum entfernbar ist,
**dadurch gekennzeichnet, dass** das zylindrische Gehäuse (4) aus durchsichtigem Material besteht und in seinem Inneren ein Ventil aufweist, das aus einem durch die Atemluft steuerbaren Kolben (5) besteht, der die dritte, in der Gebrauchslage vorzugsweise nach unten gerichtete Öffnung (3) während des Ausatmens durch den Druck der ausgeatmeten Luft und gegen den Druck einer Feder (6), seines Eigengewichts oder eines Zusatzgewichts zum Abfluß des Sputums öffnet und während des Einatmens durch den Druck der Feder (6), seines Eigengewichts oder eines Zusatzgewichts verschließt, wobei der Kolben (5) eine kegelförmige Grundfläche (7) und eine zylindrische Mantelfläche (8) aufweist, in dem zylindrischen Gehäuse (4) leicht gleitend koaxial gelagert ist und die dritte Öffnung (3) im verschlossenen Zustand mit der Mantelfläche (8) überdeckt, während die zweite, in der Gebrauchslage vorzugsweise nach oben gerichtete, in der Mantelfläche des Zylinders angeordnete Öffnung (2) immer offen ist und mit der ersten Öffnung (1) für den freien Durchgang der Atemluft in Verbindung steht.

2. Vorrichtung nach Anspruch 1, **dadurch gekennzeichnet, dass** die Öffnungen (1, 2, 3) mit zylindrisch geformten Anschlußstücken (11, 12, 13) versehen sind.

3. Vorrichtung nach Anspruch 2, **dadurch gekennzeichnet, dass** die Innenwand des Anschlußstückes (11) der ersten Öffnung (1) konisch geformt und auf das aus der Luftröhre ragende Ende des Luftröhrenkatheters aufsteckbar ist.

4. Vorrichtung nach einem der Ansprüche 2 und 3, **dadurch gekennzeichnet, dass** das Anschlußstück (12) der zweiten Öffnung (2) eine konisch geformte Außenwand aufweist, auf die ein Luftfilter für die einzuatmende Luft aufsteckbar ist.

5. Vorrichtung nach einem der Ansprüche 2 bis 4, **dadurch gekennzeichnet, dass** das Anschlußstück (13) der dritten Öffnung (3) eine konisch geformte Außenwand aufweist, auf die ein Auffangbeutel zum Auffangen des Sputums oder ein Schlauch für den Abfluß des Sputums zu einem derartigen Auffangbeutel aufsteckbar ist.

6. Vorrichtung nach einem der Ansprüche 1 bis 5, **dadurch gekennzeichnet, dass** innerhalb der zylindrischen Mantelfläche (8) des Kolbens (5) ein koaxial verlaufendes, die als Schraubenfeder ausgebildete Feder (6) in sich aufnehmendes zylindrisches Rohr (9) einstückig angeformt ist.

7. Vorrichtung nach einem der Ansprüche 1 bis 6, **dadurch gekennzeichnet, dass** das zylindrische Gehäuse (4) an der der ersten Öffnung (1) gegenüberliegenden Seite eine vierte Öffnung aufweist, die durch eine Verschlußplatte (10) verschließbar ist, an der zwei koaxial zueinander angeordnete zylindrische Rohre (14, 15) einstückig angeformt sind, wobei das äußere Rohr (14) an der Innenwand des Gehäuses (4) anliegend in das Gehäuse (4) einführbar und in eine Innenwandrille (16) einrastbar ist, während das innere Rohr (15) in die als Schraubenfeder ausgebildete Feder (6) führend eingreift.

8. Vorrichtung nach einem der Ansprüche 1 bis 7, **dadurch gekennzeichnet, dass** das Gehäuse (4) mit seinen Anschlußstücken (11, 12, 13) und/oder die in seinem Inneren angeordneten Teile (5, 6, 7, 8, 9, 10, 14, 15, 16) zumindest teilweise aus Kunststoff bestehen.

## Claims

1. A device for removing sputum from a tracheal catheter that is inserted into the trachea of a patient, said device comprising a cylindrical housing (4) with a first opening (1) inserted into the base of the cylinder, which can be connected to the tracheal catheter end that projects from the trachea, with a second opening (2) for exchanging exhaled air against filtered fresh air and with a third opening (3), arranged in the shell of the cylinder, for removing the sputum,
**characterized in that** the cylinder-shaped housing (4) is made of a transparent material and contains on the inside a valve consisting of a piston (5) controlled by respiratory air that opens during the exhalation the third opening (3), preferably downward pointing during use, as a result of the pressure exerted by the exhaled air and counter to the pressure of a spring (6), its own weight or an additional weight for draining the sputum and which closes this opening during the inhalation owing to the pressure of the spring (6), its own weight or an additional weight, said piston (5) having a cone-shaped base (7) and a cylindrical shell (8), is positioned coaxial and such that it can glide easily inside the cylindrical housing (4) and with its shell surface (8) covers the third opening (3) in the closed condition, while the second opening (2), which preferably points upward in the use position and is arranged in the shell of the cylinder, is always open and is connected to the first opening (1) for a free flow of respiratory air.

2. A device according to claim 1, **characterized in that** the openings (1, 2, 3) are provided with cylinder-shaped connecting pieces (11, 12, 13).

3. A device according to claim 2, **characterized in that** the inside wall of the connecting piece (11) for the first opening (1) has a conical shape and can be fitted onto the tracheal catheter end that projects from the trachea.

4. A device according to one of the claims 2 and 3, **characterized in that** the connecting piece (12) for the second opening (2) has a conical outside wall for fitting on an air filter for the air to be inhaled.

5. A device according to one of the claims 2 to 4, **characterized in that** the connecting piece (13) for the third opening (3) has a conical outside wall for fitting on a bag, designed to catch the sputum, or a hose for draining the sputum to a catch bag of this type.

6. A device according to one of the claims 1 to 5, **characterized in that** a coaxially extending cylindrical tube (9) is integrally formed onto the inside of the cylindrical shell (8) for piston (5), which tube accommodates the spring (6) that is designed as helical spring.

7. A device according to one of the claims 1 to 6, **characterized in that** the cylindrical housing (4) is provided with a fourth opening on the side opposite the first opening (1), which fourth opening can be closed off with a plate (10), said plate having two integrally formed-on, cylinder-shaped tubes (14, 15) that are arranged coaxial to each other, wherein the outer tube (14) can be inserted while making contact with the housing (4) inside wall and can be snapped into an inside wall groove (16) while the inner tube (15) engages in the spring (6) that is designed as helical spring and functions as a guide.

8. A device according to one of the claims 1 to 7, **characterized in that** the housing (4) with its connecting pieces (11, 12, 13) and/or the parts arranged on the inside (5, 6, 7, 8, 9, 10, 14, 15, 16) are at least in part made of a plastic and preferably of a transparent plastic.

## Revendications

1. Dispositif pour retirer des mucosités d'un cathéter trachéen introduit dans la trachée-artère d'un patient, constitué d'un boîtier cylindrique (4) présentant une première ouverture (1) ménagée dans la surface de base du cylindre, qui peut être raccordée à l'extrémité du cathéter trachéen sortant de la trachée-artère, une deuxième ouverture (2), par laquelle l'air expiré peut être échangé contre de l'air frais filtré, et une troisième ouverture, ménagée dans la surface enveloppe du cylindre, et par laquelle les mucosités peuvent être retirées,
**caractérisé en ce que** le boîtier cylindrique (4) est constitué d'un matériau transparent et présente, dans sa partie interne, une soupape qui est constituée d'un piston (5) actionnable par l'air de respiration, qui ouvre la troisième ouverture (3) dirigée de préférence vers le bas en position d'utilisation au cours de l'expiration sous la pression de l'air expiré et à l'encontre de la pression d'un ressort (6), de son poids propre ou d'un poids d'appoint pour évacuer les mucosités et la ferme au cours de l'inspiration sous l'action du ressort (6), de son propre poids ou d'un point d'appoint, le piston (5) présentant une surface de base conique (7) et une surface enveloppe cylindrique (8), étant monté coaxialement dans le boîtier cylindrique (4) de façon à coulisser aisément et recouvrant la troisième ouverture (3) à l'état fermé avec la surface enveloppe (8), tandis que la deuxième ouverture (2) ménagée dans la surface enveloppe du cylindre et dirigée de préférence vers le haut en position d'utilisation est toujours ouverte et est en communication avec la première ouverture (1) pour l'accès libre de l'air de respiration.

2. Dispositif selon la revendication 1, **caractérisé en ce que** les ouvertures (1, 2, 3) sont pourvues d'embouts (11, 12, 13) de forme cylindrique.

3. Dispositif selon la revendication 2, **caractérisé en ce que** la paroi interne de l'embout (11) de la première ouverture (1) a une forme conique et peut être enfichée sur l'extrémité du cathéter trachéen sortant de la trachée-artère.

4. Dispositif selon l'une quelconque des revendications 2 et 3, **caractérisé en ce que** l'embout (12) de la deuxième ouverture (2) présente une paroi externe de forme conique, sur laquelle peut être enfiché un filtre pour l'air à respirer.

5. Dispositif selon l'une quelconque des revendications 2 à 4, **caractérisé en ce que** l'embout (13) de la troisième ouverture (3) présente une paroi externe de forme conique, sur laquelle peut être enfiché un sac collecteur pour recevoir les mucosités ou un tube pour l'évacuation des mucosités vers un sac collecteur de ce type.

6. Dispositif selon l'une quelconque des revendications 1 à 5, **caractérisé en ce que**, à l'intérieur de la surface enveloppe cylindrique (8) du piston (5) est façonné d'une seule pièce un tube cylindrique (9) s'étendant coaxialement et recevant en soi un ressort (6) conformé en ressort hélicoïdal.

7. Dispositif selon l'une quelconque des revendications 1 à 6, **caractérisé en ce que** le boîtier cylindrique (4) présente, sur le côté opposé à la première ouverture (1), une quatrième ouverture qui peut être fermée par une plaque de fermeture (10), sur laquelle deux tubes cylindriques (14, 15) agencés coaxialement l'un par rapport à l'autre sont façonnés d'une seule pièce, le tube externe (14) adjacent à la paroi interne du boîtier (4) pouvant être guidé dans le boîtier (4) et encliqueté dans une rainure (16) de la paroi interne, tandis que le tube interne (15) s'engage en se logeant dans le ressort (6) conformé en ressort hélicoïdal.

8. Dispositif selon l'une quelconque des revendications 1 à 7, **caractérisé en ce que** le boîtier (4) avec ses embouts (11, 12, 13) et/ou les parties (5, 6, 7, 8, 9, 10, 14, 15, 16) qui sont agencées à l'intérieur du boîtier sont constitués au moins en partie d'un matériau plastique.
